# EUROPEAN PATENT SPECIFICATION

(11) **EP 2 043 537 B1**
(45) Date of publication and mention of the grant of the patent: **02.02.2011**
(21) Application number: 07804542.4
(22) Date of filing: 27.06.2007
(51) Int. Cl.: A61B 17/86, A61B 17/68

(54) **BONE SCREW**
KNOCHENSCHRAUBE
VIS À OS

(30) Priority: 05.07.2006 FR 0606100
(43) Date of publication of application: 08.04.2009
(73) Proprietor: Implants International Limited, Cleveland Thornaby-on-Tees TS17 9LZ (GB)
(72) Inventor: EMMANUEL, Mohan, Station Town Durham TS28 5NA (GB)
(74) Representative: Jeannet, Olivier
(86) International application number: PCT/IB2007/001771
(87) International publication number: WO 2008/004057

(56) References cited:
- EP-A2- 1 306 058
- US-A- 4 013 071

## Description

The present invention relates to a bone screw, in particular a screw intended to fix, with other screws, a cortical plate, in particular a plate for immobilizing cervical vertebrae.

Cortical plates are currently fixed using a screw. In some cases, a significant problem concerning the screws' ability to hold may occur with regard to the bones in question, particularly when these bones are cervical vertebrae. Indeed, the insertion areas for the screws are reduced in this case and the repeated stresses exerted on the cervical vertebrae generate a significant risk of unscrewing for the screws.

It is known to equip cortical plates with covering parts intended to cover the heads of the screws after installation, or to anticipate inter-engagement means of the screw heads with areas of the plates defining insertion holes for the screws.

It is also known to use "expansion" screws; a screw of this type comprises a tubular screw body defining an axial cavity, at least one portion of which comprises transverse radial slots, and a rod intended to be screwed into said cavity to cause expansion of the portions of the screw body defined by said slots.

The screws of the prior art have the drawback of not being very easy to use, requiring the placement of the body of the screw, then the placement of the rod, which leads to substantial difficulties. Immobilization of the rod relative to the screw body may by uncertain in some cases.

Document EP 1 306 058 describes an expansion screw of this type in which the expansion rod is not permanently contained inside the body of the screw; this expansion rod comprises a head being closely fitted in a proximal cavity formed by the screw body, to screw locking projections comprised by the portions of this head into the corresponding recesses located in the holes of the plate.

The screw according to this prior document does not resolve the aforementioned drawbacks.

The present invention aims to resolve these drawbacks.

The invention relates to an "expansion" screw, comprising a tubular screw body defining an axial cavity, which forms an expandable head comprising first transverse radial slots, and an expansion rod able to be moved in said cavity so as to cause an expansion of the portions of the screw body defined by said slots, the screw body and expansion rod comprising threaded portions to allow movement of the expansion rod in the screw body.

According to the invention,
- the cavity of the screw body presents a portion with an enlarged cross-section forming a chamber, positioned such that it is set back from the proximal surface of said head, over a sufficient distance so that said first slots and said first expandable portions which define these first slots constitute gripping means to drive the screw head in rotation;
- said expansion rod comprises a proximal head equipped with gripping means to drive it in rotation, intended to be found in said chamber in the non-expanded position of the screw head;
- the cavity of the screw body has a first portion with a limited cross-section located at the proximal zone of said first expandable portions, this limited cross-section portion being intended to cooperate with the proximal head of the expansion rod to achieve expansion of said first expandable portions while also defining a passageway having a cross-section sufficient to allow access, from the outside of the screw, to the gripping means comprised by the expansion rod.

Thus, in the unexpanded position, the head of the expansion rod is sufficiently withdrawn from the head of the screw to avoid hindering gripping of the screw by a suitable tool, thanks to the gripping means constituted by said first slots and said first expandable walls; the expansion screw may, however, be maneuvered through said first limited cross-section portion so as to be able to be brought into the expanded position of said first expandable walls.

Consequently, the screw contains the expansion rod from before installation and may be used as a traditional screw when it is installed; once the screw has been installed, the expansion rod may be withdrawn by unscrewing such that its head cooperates with said first limited cross-section portion.

Preferably, said proximal head or the surface of said first limited cross-section area, or both, comprise(s) a surface appearance intended to prevent sliding of said proximal head with regard to said first limited cross-section area when this head is located at this first limited cross-section area.

This surface appearance makes it possible to encourage rotational locking of the proximal head relative to the first limited cross-section area, such that the expanded position of the screw is perfectly maintained, notwithstanding the repeated stresses which may be exerted on this screw.

According to a preferred embodiment, in this case, the surface of said first limited cross-section portion is corrugated.

Advantageously,
- the body of the screw comprises an expandable distal portion, meaning second slots opening in the distal end of this screw body, defining second expandable portions;
- the cavity of the screw body and the distal portion of the rod present respective portions intended to cooperate to achieve the expansion of said second expandable portions when said proximal head comes into contact with said first limited cross-section portion.

According to one preferred embodiment of the invention, in this case,
- the cavity of the screw body presents a second limited cross-section portion located at the distal zone of said second expandable portions, and
- said expansion rod comprises a limited cross-section area intended to be found, in the unexpanded position, at the level of said second limited cross-section portion, and a distal zone having an enlarged cross-section relative to the cross-section of this limited cross-section zone, to carry out the expansion of said second expandable portions.

The distal end of said expansion rod may form a tip developed such that it protrudes from the distal end of the screw body in the unexpanded position.

This tip facilitates insertion of the screw into a bone.

The invention shall be well understood, and other characteristics and advantages thereof shall appear, in reference to the annexed diagrammatic drawing, illustrating, non-exhaustively, one preferred embodiment of the screw to which it relates.

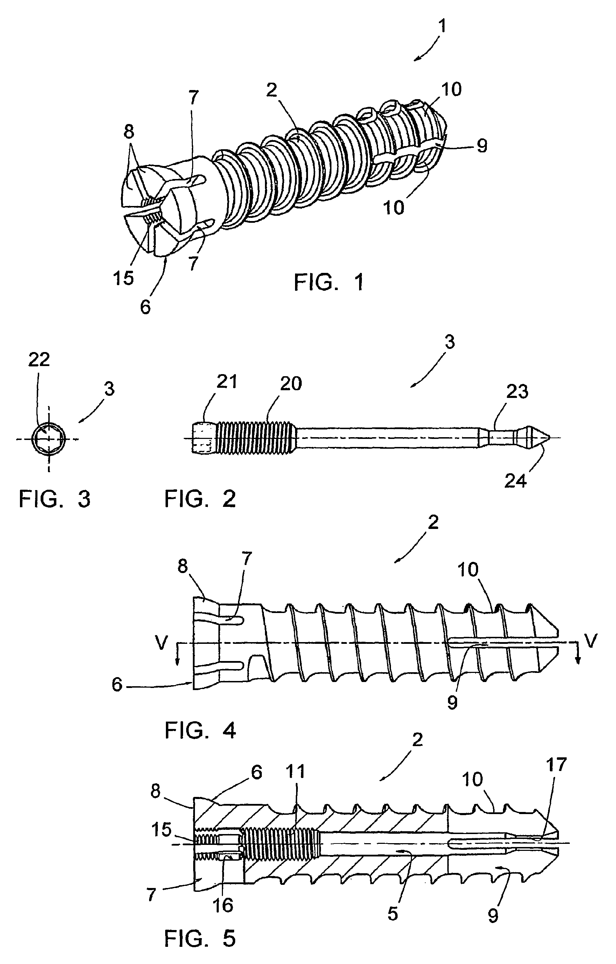

Figure 1 is a perspective view of the invention;
figure 2 is a side view of an expansion rod comprised by the invention;
figure 3 is a view of this expansion rod, at the end, proximal side;
figure 4 is a side view of the screw body comprised by the screw according to the invention;
figure 5 is a view of this screw body in cross-section according to line V-V of figure 4;
figure 6 is a view similar to figure 5, after installation of the expansion rod in the screw body, in an unexpanded position;
figure 7 is an end view of the screw, proximal side;
figure 8 is an end view of the screw, distal side, and
figure 9 is a view similar to figure 6, in an expanded position.

The figures illustrate a bone screw 1, particularly intended to fix, with other screws, a cortical plate, in particular a plate for immobilizing cervical vertebrae.

The screw 1 comprises a screw body 2 and an expansion rod 3.

The body 2 is tubular and defines an axial cavity 5. It forms a head 6 at its proximal end, comprising five transverse radial slots 7 which open into its proximal surface, these slots 7 thereby defining expandable portions 8; the body 2 also comprises four transverse radial slots 9 located in its distal portion, which open at its distal end, these slots 9 thereby also defining expandable portions 10.

The body 2 also comprises a threaded portion 11, with which a threaded portion 20 presented by the expansion rod 3 is intended to cooperate.

On the proximal side, the cavity 5 presents a portion 15 having a limited cross-section and a portion 16 having an enlarged cross-section forming a chamber.

The portion 15 is located at the proximal area of the expandable portions 8; it presents a limited cross-section such that it can cooperate with a proximal head 21 comprised by the expansion rod 3 to achieve expansion of the portions 8, while also defining a passage having a cross-section sufficient to allow access, from the outside of the screw 1, to a proximal hexagonal cavity 22 comprised by the expansion rod 3 for its screwing or unscrewing in the body 2. The portion 15 is moreover corrugated and thus has a non-smooth surface appearance, so as to prevent sliding of the proximal head 21 of the expansion rod 3 relative to it when this head 21 is located at the corrugated surface of the portion 15.

The portion 16 is positioned withdrawn from the proximal surface of the head 6, between the portions 15 and 11 in the illustrated example. It has a cross-section slightly larger than that of the head 21 of the expansion rod 3.

From the distal side, the body 2 comprises a portion 17 having a limited cross-section, developed in the distal areas of the expandable portions 10. The portion 17 is axially bordered by slanted areas forming ramps.

The expansion rod 3 comprises, beyond the threaded portion 20, the aforementioned proximal head 21 and hexagonal cavity 22, a distal portion 23 having a limited cross-section, axially bordered by slanted forming ramps, and a pointed distal end 24.

The expansion rod 3 is intended to be engaged inside the body 2 until engagement of the portions 20 and 11, then engagement of the head 21 in said chamber. In this position, which is the unexpanded position of the screw 1, the head 21 finds itself at a sufficient distance from the proximal surface of the screw 1 for said slots 7 and the expandable portions 8 to constitute gripping means to drive the screw 1 in rotation using a suitable tool. In this same position, the area 23 of the rod 3 finds itself facing the portion 17 of the body 2, this area 23 and this portion 17 being sized such that no expansion of the portions 10 takes place. Moreover, the pointed distal end 24 of the rod 3 protrudes beyond the distal end of the body 2.

It should be noted that the threaded portion 20 presents a length such that it engages with the thread of the portion 11 when the head 21 is not in contact with the portion 15. Crossing of this portion 15 by this head 21, against the elastic return force of the portions 8, is thus easily achieved, by simple screwing of the rod 3 into the body 2.

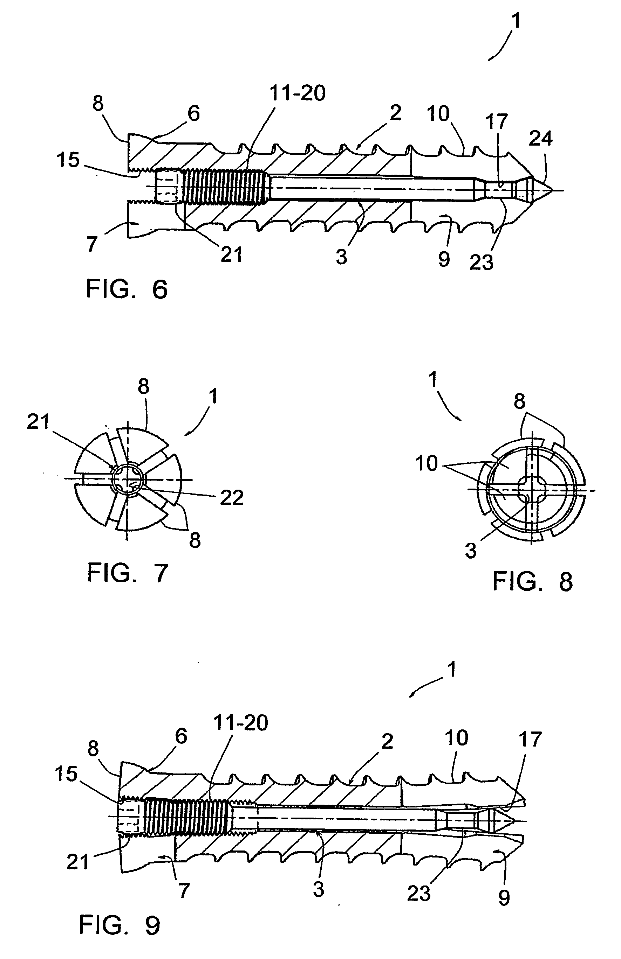

As shown more particularly in figure 7, the cross-section of the passageway delimiting the portion 15 remains sufficient to allow maneuvering of the expansion rod 3 through the portion 15, so as to be able to unscrew the rod 3 toward the position illustrated in figure 9. In this position, which is the expanded position of the screw 1, the head 21 arrives at the portion 15 and carries out the expansion of the portions 8, and the distal end of the rod 3 comes across from the portion 17, which carries out the expansion of the portions 10.

As shown by the preceding, the invention provides a bone screw very easily used given that it does not require installation of the body 2 and then the rod 3, and that immobilization of the rod 3 relative to the body 2 is perfectly provided.

It goes without saying that the invention is not limited to the embodiment described above as an example, but that it extends to all embodiments covered by the annexed claims.

## Claims

1. Bone screw (1), of type "expansion" screw, comprising a tubular screw body (2) defining an axial cavity (5), which forms an expandable head (6) comprising first transverse radial slots (7), and an expansion rod (3) able to be moved in said cavity (5) so as to cause an expansion of the portions (8) of the screw body (2) defined by said slots (7), the screw body (2) and expansion rod (3) comprising threaded portions (11, 20) to allow movement of the expansion rod (3) in the screw body (2), **characterized in that**:
- the cavity (5) of the screw body (2) presents a portion with an enlarged cross-section (16) forming a chamber, positioned such that it is set back from the proximal surface of said head (6), over a sufficient distance so that said first slots (7) and said first expandable portions (8) which define these first slots (7) constitute gripping means to drive the screw (1) in rotation;
- said expansion rod (3) comprises a proximal head (21) equipped with gripping means (22) to drive it in rotation, intended to be found in said chamber in the non-expanded position of the screw head (6);
- the cavity (5) of the screw body (2) has a first portion (15) with a limited cross-section located at the proximal zone of said first expandable portions (8), this limited cross-section portion (15) being intended to cooperate with the proximal head (21) of the expansion rod (3) to achieve expansion of said first expandable portions (8) while also defining a passageway having a cross-section sufficient to allow access, from the outside of the screw (1), to the gripping means (22) comprised by the expansion rod (3).

2. Screw (1) according to claim 1, **characterized in that** said proximal head (21) or the surface of said first limited cross-section area (15), or both, comprise(s) a surface appearance intended to prevent sliding of said proximal head (21) with regard to said first limited cross-section area (15) when this head (21) is located at this first limited cross-section area (15).

3. Screw (1) according to claim 2, **characterized in that** the surface of said first limited cross-section portion (15) is corrugated.

4. Screw (1) according to any of claims 1 to 3, **characterized in that**:
- the body (2) of the screw comprises an expandable distal portion, meaning second slots (9) opening in the distal end of this screw body (2), defining second expandable portions (10);
- the cavity (5) of the screw body (2) and the distal portion of the rod present respective portions (17, 23) intended to cooperate to achieve the expansion of said second expandable portions (10) when said proximal head (21) comes into contact with said first limited cross-section portion (15).

5. Screw (1) according to claim 4, **characterized in that**:
- the cavity (5) of the screw body (2) presents a second limited cross-section portion (17) located at the distal zone of said second expandable portions (10), and
- said expansion rod (3) comprises a limited cross-section area (23) intended to be found, in the unexpanded position, at the level of said second limited cross-section portion (17), and a distal zone having an enlarged cross-section relative to the cross-section of this limited cross-section zone (23), to carry out the expansion of said second expandable portions (10).

6. Screw (1) according to any of claims 1 to 5, **characterized in that** the distal end of said expansion rod (3) forms a tip developed such that it protrudes from the distal end of the screw body (2) in the unexpanded position.

7. Screw (1) according to any of claims 1 to 6, **characterized in that** the threaded portion (20) of the expansion rod (3) presents a length such that, at the moment of the installation of the expansion rod (3) in the screw body (2), the engagement of this threaded portion (20) with the threaded portion (11) of the body screw (2) intervenes before said proximal head (21) meet said first limited cross-section portion (15).

## Patentansprüche

1. Knochenschraube (1), vom Typ "Expansionschraube", mit einem rohrförmigen Schraubekörper (2), der einen axialen Hohlraum (5) begrenzt, der einen erweiterbaren Kopf (6) bildet, dieser Kopf umfassend ersten quer Radialschlitze (7) und eine Expansionstange (3), die sich in diesem Hohlraum (5) bewegen kann, so dass es eine Erweiterung der Teile (8) des Schraubekörpers (2), der durch diese Schlitze (7) begrenzt ist; auswirkt; der Schraubekörper (2) und die Expansionstange (3) umfassend Gewindeportions (11, 20) um die Bewegung der Expansionstange (3) in die Schraubekörper (2) zu ermöglichen, wobei die Knochenschraube **dadurch gekennzeichnet ist, dass**:
- der Hohlraum (5) des Schraubekörpers (2) einen Teil mit einem vergrößerten Querschnitt (16) aufweist, der eine Kammer bildet, die so ausgerichtet ist, dass es sich von der proximalen Seite des Kopfes (6) in Rücktritt befindet, über einen ausreichenden Abstand, so dass die ersten Schlitze (7) und die ersten erweiterbaren Teile (8), die diese ersten Schlitze begrenzen (7), Greifmitteln für den Antrieb der Schraube (1) in Rotation bilden;
- die Expansionstange (3) einen proximalen Kopf (21) aufweist, der mit Greifmitteln (22) ausgestattet ist, für seinen Antrieb in Rotation; diese Expansionstange ist vorgesehen, sich in Position von nicht-expandierten des Schraubenkopfes (6) in der Kammer zu befinden;
- der Hohlraum (5) des Schraubekörpers (2) einen ersten Teil (15) mit einem begrenzten Querschnitt an der proximalen Zone der ersten erweiterbaren Teile (8) aufweist; dieser Teil (15) ist mit einem begrenzten Querschnitt vorgesehen, mit dem proximalen Kopf (21) der Expansionstange (3) zu kooperieren, um die Expansion der ersten erweiterbaren Teile (8) aufzuführen, dieser Teil (15) begrenzend darüberhinaus einen Durchgang mit einem aufreichenden Querschnitt, um den Zugang von außerhalb der Schraube (1) nach den Greifmitteln (22) der Expansionstange (3) zu ermöglichen.

2. Schraube (1) nach Anspruch 1, **dadurch gekennzeichnet, dass** der proximale Kopf (21) oder die Seite dieses ersten Teiles (15) mit begrenzten Querschnitt, oder beides, ein Aussehen der Seite umfasst (umfassen) zur Verhinderung Gleiten von diesem proximalen Kopf (21) im Verhältnis zu diesem ersten Teil (15) mit begrenzten Querschnitt, wenn dieser Kopf (21) in diesem ersten Teil (15) mit begrenzten Querschnitt sich befindet.

3. Schraube (1) nach Anspruch 2, **dadurch gekennzeichnet, dass** die Seite dieserm Teil (15) mit begrenzten Querschnitt gewellt ist.

4. Schraube (1) nach einem der Ansprüche 1 bis 3, **dadurch gekennzeichnet, dass**:
- der Körper (2) der Schraube einen erweiterbaren distalen Teil umfasst, das heißt zweiten Schlitze (9), die sich in dem distalen Ende dieser Schraubekörper (2) münden, dieser Teil abgrenzend zweiten ausdehnbaren Teile (10);
- der Hohlraum (5) des Schraubekörpers (2) und der distale Teil der Stange jeweiligen Teile (17, 23) aufweisen, die geeignet sind, zu kooperieren, um, während der Kontaktierung des Kopfes (21) mit diesem ersten Teil (15) mit begrenzten Querschnitt, die Expansion von der zweiten erweiterbaren Teile (10) zu erreichen.

5. Schraube (1) nach Anspruch 4, **dadurch gekennzeichnet, dass**:
- der Hohlraum (5) des Schraubekörpers (2) einen zweiten Teil (17) mit einem begrenzten Querschnitt aufweist, der an der distalen Zone der zweiten erweiterbaren Teile (10) sich befindet, und
- diese Expansionstange (3) einen Bereich (23) mit einem begrenzten Querschnitt aufweist, die vorgesehen ist, sich in der nicht-expandierten Position auf der Ebene den zweiten Teil (17) mit begrenzten Querschnitt zu befinden; diese Expansionstange aufweisend darüberhinaus eine distale Zone, die einen vergrößerten Querschnitt im Verhältnis zu diesem Bereich (23) mit begrenzten Querschnitt umfasst, um die Expansion des zweiten erweiterbaren Teiles (10) aufzuführen.

6. Schraube (1) nach einem der Ansprüche 1 bis 5, **dadurch gekennzeichnet, dass** das distale Ende der Expansionstange (3) eine Spitze bildet, die entwickelt ist, so dass es aus dem distalen Ende des Schraubekörpers (2) in der expandierten Position ragt.

7. Schraube (1) nach einem der Ansprüche 1 bis 6, **dadurch gekennzeichnet, dass** die Gewindeportion (20) der Expansionstange (3) eine Länge aufweist, so dass, während der Bereitstellung der Expansionstange (3) in dem Schraubekörper (2), und bevor, dass der proximale Kopf (21) den ersten Teil (15) mit begrenzten Querschnitt auftrifft, findet der Eingriff dieser Gewindeportion (20) mit der Gewindeportion (11) des Schraubekörpers (2) statt.

## Revendications

1. Vis (1) à os, du type "à expansion", comprenant un corps (2) de vis tubulaire délimitant une cavité axiale (5), qui forme une tête expansible (6) comprenant des premières fentes radiales traversantes (7), et une tige d'expansion (3) pouvant être déplacée dans ladite cavité (5) de manière à provoquer une expansion des portions (8) du corps (2) de vis délimitées par lesdites fentes (7), le corps (2) de vis et la tige d'expansion (3) comprenant des portions filetées (11, 20) pour permettre le déplacement de la tige d'expansion (3) dans le corps (2) de vis, **caractérisée en ce que** :
- la cavité (5) du corps (2) de vis présente une portion de section élargie (16) formant une chambre, aménagée en retrait de la face proximale de ladite tête (6), sur une distance suffisante pour que lesdites premières fentes (7) et lesdites premières portions expansibles (8) que délimitent ces premières fentes (7) constituent des moyens de prise pour l'entraînement de la vis (1) en rotation ;
- ladite tige d'expansion (3) comprend une tête proximale (21) équipée de moyens de prise (22) pour son entraînement en rotation, destinée à se trouver dans ladite chambre en position de non expansion de la tête (6) de vis ;
- la cavité (5) du corps (2) de vis présente une première portion (15) de section restreinte aménagée au niveau de la zone proximale desdites premières portions expansibles (8), cette première portion (15) de section restreinte étant destinée à coopérer avec la tête proximale (21) de la tige d'expansion (3) pour réaliser l'expansion desdites premières portions expansibles (8) tout en délimitant également un passage de section suffisante pour permettre l'accès, depuis l'extérieur de la vis (1), aux moyens de prise (22) que comprend la tige d'expansion (3).

2. Vis (1) selon la revendication 1, **caractérisée en ce que** ladite tête proximale (21) ou la surface de ladite première zone (15) de section restreinte, ou les deux, comprend (comprennent) un aspect de surface propre à empêcher le glissement de ladite tête proximale (21) par rapport à ladite première zone (15) de section restreinte lorsque cette tête (21) se trouve au niveau de cette première zone (15) de section restreinte.

3. Vis (1) selon la revendication 2, **caractérisée en ce que** la surface de ladite première portion (15) de section restreinte est striée.

4. Vis (1) selon l'une quelconque des revendications 1 à 3, **caractérisée en ce que**:
- le corps (2) de vis comprend une portion distale expansible, c'est-à-dire des deuxièmes fentes (9) débouchant dans l'extrémité distale de ce corps (2) de vis, délimitant des deuxièmes portions expansibles (10) ;
- la cavité (5) du corps (2) de vis et la portion distale de la tige présentent des portions respectives (17, 23) propres à coopérer pour réaliser l'expansion desdites deuxièmes portions expansibles (10) lors de la venue en coopération de ladite tête proximale (21) avec ladite première portion (15) de section restreinte.

5. Vis (1) selon la revendication 4, **caractérisée en ce que** :
- la cavité (5) du corps (2) de vis présente une deuxième portion (17) de section restreinte aménagée au niveau de la zone distale desdites deuxièmes portions expansibles (10), et
- ladite tige d'expansion (3) comprend une zone (23) de section restreinte destinée à se trouver, en position de non expansion, au niveau de ladite deuxième portion (17) de section restreinte, et une zone distale de section élargie par rapport à la section de cette zone (23) de section restreinte, pour réaliser l'expansion desdites deuxièmes portions expansibles (10).

6. Vis (1) selon l'une quelconque des revendications 1 à 5, **caractérisée en ce que** l'extrémité distale de ladite tige d'expansion (3) forme une pointe aménagée de manière à faire saillie de l'extrémité distale du corps (2) de vis en position de non expansion.

7. Vis (1) selon l'une des revendications 1 à 6, **caractérisée en ce que** la portion filetée (20) de la tige d'expansion (3) présente une longueur telle que, lors de la mise en place de la tige d'expansion (3) dans le corps (2) de vis, la venue en prise de cette portion filetée (20) avec la portion filetée (11) du corps (2) de vis intervient avant que ladite tête proximale (21) ne rencontre ladite première portion (15) de section restreinte.
